# EUROPEAN PATENT APPLICATION

(11) **EP 4 744 683 A1**
(43) Date of publication of application: **20.05.2026**
(21) Application number: 24839880.2
(22) Date of filing: 07.05.2024
(51) Int. Cl.: A61K 48/00, A61K 31/7088, A61P 37/00, A61P 9/10, A61P 35/00, A61P 11/00, A61P 9/04

(54) **THERAPEUTIC AGENT FOR HIF-1ALPHA -RELATED DISEASE USING AREL1 GENE REGULATORY OF HIF-1ALPHA ACTIVITY**

(30) Priority: 12.07.2023 KR 20230090751
(71) Applicant: Biochemgen Inc., Cheonan-si Chungcheongnam-do 31116 (KR)
(72) Inventor: SHIN, Deug-Yong, Yongin-si Gyeonggi-do 17098 (KR)
(74) Representative: dompatent
(86) International application number: PCT/KR2024/006090
(87) International publication number: WO 2025/014063

(57) **Abstract**

Disclosed is a therapeutic agent for preventing, treating or diagnosing HIF-1α-related diseases using an AREL1 gene regulating HIF-1α activity.

Provided is a HIF-1α activity modulator containing, as an active ingredient, at least one of a compound, hexane, an antibody, and a peptide that regulates expression and activity of an AREL1 (apoptosis-resistant E3 ubiquitin protein ligase 1) gene or an AREL1 protein encoded by the AREL1 gene, or that interferes with interaction between the AREL1 gene and PHD2.

## Description

### [Technical Field]

The present invention relates to a therapeutic agent for preventing, treating or diagnosing HIF-1α-related diseases using an AREL1 gene regulating HIF-1α activity.

### [Background Art]

The present research team has previously published a patent (Korean Patent No. 10-1274256) and a paper relating to the AREL1 gene as an apoptosis regulator. The prior patent referred to the name of the gene as RANI1, but prior to publication of the paper, according to the recommendation of the HUGO Gene Nomenclature Committee (HGNC), the name was changed to AREL1 (apoptosis-resistant E3 ubiquitin protein ligase 1), and was registered with HUGO (Human Genome Organization) under this name.

The prior patent demonstrates that the AREL1 gene suppresses apoptosis by inducing ubiquitination and degradation of apoptosis regulators. Based on this, the efficacies of a therapeutic agent for treating apoptosis-related diseases, such as inducing apoptosis in chemotherapeutic-resistant cancer cells, inhibiting apoptosis in cardiomyocytes during cardiovascular disease, and inhibiting apoptosis in surrounding brain cells due to stroke were demonstrated. However, the present invention aims at proving that AREL1 regulates the HIF-1α activation function and the expression and functions of the HIF-1α target genes, thereby having efficacy as a therapeutic agent for diseases caused by HIF-1α.

### [Disclosure]

### [Technical Problem]

Therefore, the present invention has been made in view of the above problems, and it is one object of the present invention to demonstrate that HIF-1α is increased and activated by ubiquitination and degradation of PHD2 by the expression of AREL1. As a result, it is another object of the present invention to provide a composition for treating an HIF-1α-related disease, in which HIF-1α is the cause or the regulation of HIF-1α activity is related to the occurrence and treatment of the disease, using the AREL1 gene that regulates the activity of HIF-1α (1) by proposing AREL1 as a regulatory gene for HIF-1α activity, and (2) by proposing an AREL1 expression or activity regulator, or an AREL1-PHD2 interaction regulator as a regulator for HIF-1α activity.

Here, HIF-1α is a transcription factor that plays an essential role in cell adaptation to hypoxia. However, HIF-1α activation causes fatal diseases, for example, immune diseases such as autoimmune diseases, ischemic/valvular heart diseases, stroke, cancer, chronic lung diseases, and congestive heart failure. For this reason, the present inventors searched for genes that regulate HIF-1α activity and researched the genes as development targets for therapeutic agents for HIF-1α-related diseases.

The objects of the present invention are not limited to that described above. Other objects not mentioned herein will be easily understood by those skilled in the art from the description below.

### [Technical Solution]

In accordance with the present invention, the above and other objects can be accomplished by the provision of a therapeutic agent for HIF-1α-related diseases using an AREL1 gene regulating HIF-1α activity containing, as an active ingredient, at least one of a compound, a hexane, an antibody, and a peptide that regulates expression and activity of an AREL1 (apoptosis-resistant E3 ubiquitin protein ligase 1) gene or an AREL1 protein encoded by the AREL1 gene, or that interferes with interaction between the AREL1 gene and PHD2.

The therapeutic agent for HIF-1α-related diseases using the AREL1 gene regulating HIF-1α activity according to the present invention is used for treatment, prevention, or diagnosis of a HIF-1α-related disease, for example, at least one disease of autoimmune disease, ischemic/valvular heart disease, stroke, cancer, chronic lung disease, and congestive heart failure.

In the therapeutic agent for HIF-1α-related diseases using the AREL1 gene regulating HIF-1α activity according to the present invention, the autoimmune disease may include at least one of rheumatoid arthritis, alopecia areata, psoriasis, type 1 diabetes, hyperthyroidism, chronic thyroiditis, hypothyroidism, vitiligo, Crohn's disease, and autoimmune infections.

Furthermore, the composition for treating HIF-1α-related diseases according to the present invention may be administered as an appropriate formulation along with a diluent or excipient. The pharmaceutical composition of the present invention may be administered via any common route as long as it can reach the target tissue. Depending on the intended method, the pharmaceutical composition may be administered parenterally (e.g., intravenously, subcutaneously, intraperitoneally, or topically) or orally. Parenteral administration is particularly preferred, and intravenous injection is more particularly preferred. The formulation varies depending on the selected administration method.

Pharmaceutically acceptable carriers include at least one of saline, sterile water, Ringer's solution, buffered saline, dextrose solution, maltodextrin solution, glycerol, ethanol, liposomes, and mixtures thereof. Other common additives, such as antioxidants, buffers, and bacteriostatic agents, may be added as needed. Furthermore, the composition may be prepared into injectable formulations, such as aqueous solutions, suspensions, and emulsions, pills, capsules, granules, or tablets by further adding diluents, dispersants, surfactants, binders, and lubricants. Target organ-specific antibodies or other ligands may be used in combined with the carrier to act specifically on the target organs. Furthermore, the composition may be suitably formulated depending on each disease or component using any appropriate method in the art or methods disclosed in Remington's Pharmaceutical Science (recent edition), Mack Publishing Company, Easton PA.

Furthermore, other pharmaceutical delivery systems, such as liposomes and emulsions, well-known in the art, may be used. Certain organic solvents, such as dimethyl sulfoxide, may also be used.

The dosage will vary depending on the weight, age, gender, health, diet, administration time, administration method, excretion rate, and disease severity of the patient. For example, a therapeutically effective dosage may be initially determined using *in vitro* assays based on cell cultures. Experiments in animal models may then be performed to determine the range of Rani concentrations in the blood using the IC₅₀ (the concentration of the test compound that is lethal to 50% of the cultured cells upon drug treatment in an *in vitro* assay) determined in cell cultures. Those skilled in the art will be able to determine a therapeutically effective dose without extensive experimentation, and this information may be used to more accurately determine a useful dosage in humans.

### [Advantageous effects]

According to the present invention, the therapeutic agent for the HIF-1α-related disease using the AREL1 gene that regulates HIF-1α activity has the effect of preventing, treating, or diagnosing diseases caused by HIF-1α using AREL1 as a regulatory gene of HIF-1α activity and using an expression or activity regulator or AREL1-PHD2 interaction regulator as a regulator of HIF-1α activity, based on the finding that HIF-1α is increased and activated by ubiquitination and degradation of PHD2 through the expression of AREL1.

The effects of the present invention are not limited to that described above. Other effects not mentioned herein will be easily understood by those skilled in the art from the description below.

### [Description of Drawings]

FIG. 1 illustrates an increase in HIF-1α due to AREL1 expression.
FIG. 2 illustrates a decrease in PHD2 and an increase in HIF-1α due to increased AREL1 expression.
FIG. 3 illustrates an increase in HIF-1α and HIF-1α target genes due to AREL1 expression.
FIG. 4 illustrates an increase in PHD2 and the decrease in HIF-1α and VEGF due to AREL1 inhibition.
FIG. 5 illustrates AREL1-PHD2 interaction.
FIG. 6 illustrates PHD2 ubiquitination due to AREL1 expression.
FIG. 7 illustrates regulation of angiogenesis by AREL1.

### [Best Mode]

Hereinafter, preferred embodiments of the present invention will be described in detail. However, the present invention is not limited to the embodiments and may be embodied in different forms. The embodiments are suggested only to offer thorough and complete understanding of the disclosed contents and to sufficiently inform those skilled in the art of the technical concept of the present invention.

### [Example 1]

### - Increase in HIF-1α by AREL1 expression

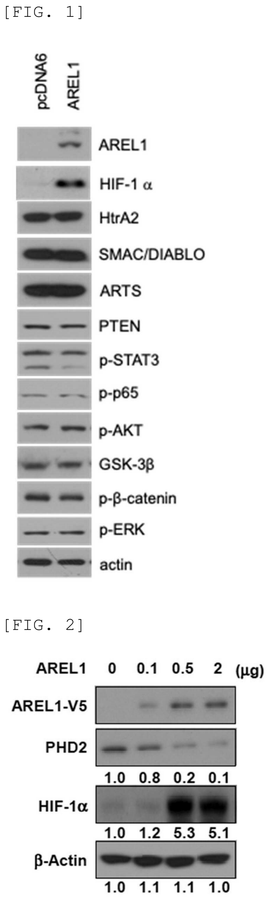

Protein was extracted from the AREL1-expressing cell line (AREL1) and the control cells (NIH3T3) prepared by introducing the AREL1 expression vector into NIH3T3 cells, and quantitative changes in protein levels were determined using Western blot analysis. The results showed that AREL1 expression increased HIF-1α (hypoxia-inducible factor-1α). This quantitative increase in HIF-1α indicates increased protein stability due to decreased degradation by PHD2 (prolyl-hydroxylase domain 2) (see FIG. 1).

That is, in order to determine which signaling pathways are activated by AREL1 expression, the AREL1 gene was introduced into NIH3T3 cells and then regulatory proteins of various signaling pathways were detected by Western blot. The results showed that AREL1 expression increased HIF-1α.

### [Example 2]

### - Decrease in PHD2 and increase in HIF-1α caused by AREL1

To determine Example 1 showing that HIF-1α was increased by AREL1, AREL1 expression vectors were introduced at doses of 0.1 µg, 0.5 µg, and 2 µg, respectively, to gradually increase AREL1 expression. The result of HIF-1α analysis showed that, as AREL1 expression increased, HIF-1α further increased (see FIG. 2).

Because HIF-1α was increased by AREL1, the possibility that PHD2 regulating HIF-1α was regulated by AREL1 was determined. The results showed that, as AREL1 increased, PHD2 decreased inversely. Therefore, this result that AREL1 expression decreased PHD2 and increased HIF-1α indicates that AREL1 activates HIF-1α based on a decrease in PHD2.

That is, as AREL1 expression increases, PHD2 further increases and HIF-1α further decreases, which means a clear correlation of AREL1-PHD2-HIF-1α.

### [Example 3]

### - Changes in expression of HIF-1α target genes due to the increase or decrease of AREL1

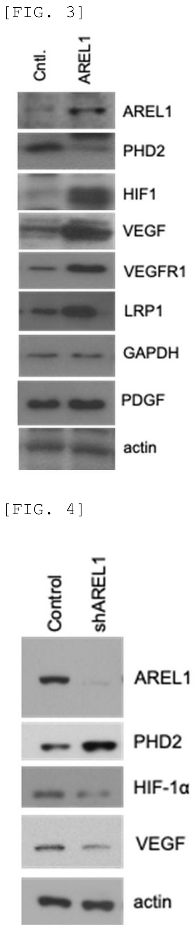

AREL1 or shAREL1 (shRNA of AREL1, AATTGGTCCCTGAGAACCTTT) was cloned into a retroviral vector (pBabe-puro) to produce viruses expressing AREL1 or shAREL1. H1299 cells were infected with these viruses to cause AREL1 or shAREL1 expression. Cells expressing AREL1 increased AREL1 expression, resulting in a decrease in PHD2 and an increase in HIF-1α. In addition, target genes of HIF-1α transcription factors, such as VEGF, increased. Therefore, an increase in HIF-1α indicates an increase in activity (see FIG. 3).

H1299 cells infected with a virus expressing shAREL1, which inhibits AREL1 expression, exhibited a decrease in AREL1. As AREL1 decreased, PHD2 increased, and HIF-1α decreased. As HIF-1α decreased, VEGF, a transcriptional target of HIF-1α, also decreased. This indicates a decrease in the transcriptional activity of HIF-1α (see FIG. 4).

### [Example 4]

### - Binding between AREL1 and PHD2

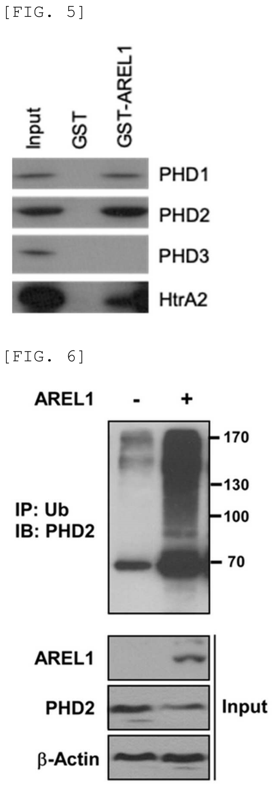

HIF-1α is hydroxylated by PHD2, and the hydroxylated HIF-1α is ubiquitinated and degraded by VHL. Since AREL1 expression increased HIF-1α, but decreased PHD2, the possibility that AREL1 binds to PHD2 to cause ubiquitination was determined. Proteins from H1299 cells were reacted with GST or GST-AREL1 and then proteins bound to GST or GST-AREL1 were collected and analyzed by Western blotting. The result showed that HtrA2, which is a protein that binds to AREL1, was identified and used as a positive control. Whether or not antibodies of the three PHD isoforms bound to AREL1 was determined. The result showed that PHD1/2 bound to AREL1, while PHD3 did not bind to AREL1. Therefore, PHD2, which plays a crucial role in HIF-1α regulation, binds to AREL1 (see FIG. 5).

### [Example 5]

### - Ubiquitination and degradation of PHD2 by AREL1

Whether or not increased AREL1 expression leads to increased PHD2 ubiquitination was determined based on the results that AREL1 expression decreases PHD2 levels and that AREL1 binds to PHD2 in the cells.

Proteins were extracted from AREL1-expressing HCT116 cancer cells and control cells, were immunoprecipitated with a ubiquitin (Ub) antibody, and then Western blotting was performed with a PHD2 antibody. AREL1 expression increases PHD2 ubiquitination, resulting in large-molecular-weight PHD2 in a ladder-like shape. To verify the amount of protein input in the experiment, an amount of protein identical to that used for immunoprecipitation was subjected to Western blotting with AREL1, PHD2, and actin antibodies, and used as an input control.

That is, AREL1 or PHD2 expression vectors were introduced into HCT116 cells to overexpress each gene. After immunoprecipitation with a ubiquitin (Ub) antibody, the cells were then subjected to western blotting with a PHD2 antibody. An increase in PHD2 ubiquitination was observed in AREL1 cells compared to the control group (see FIG. 6). This result shows a mechanism by which AREL1 activates HIF-1α, which is inhibited by PHD2, by inducing degradation of PHD2 through ubiquitination of PHD2.

### [Example 6]

### - Increased PHD2, decreased HIF-1α, and decreased angiogenesis due to AREL1 inhibition

HIF-1α activity is regulated by increased or decreased AREL1 expression. Therefore, whether or not HIF-1α-induced angiogenesis is regulated by increased or decreased AREL1 expression was determined using HUVEC assay.

For this purpose, H1299 cells expressing AREL1 or shAREL1, as used in Example 3, were used. Vascular endothelial HUVEC cells were seeded on Metrigel-coated plates and cultured in the culture medium containing H1299 cells, AREL1-expressing cells, and shAREL1-expressing cells. After 24 hours, capillary tube formation is shown in the drawing. To quantify this, one tube was considered as three branch points.

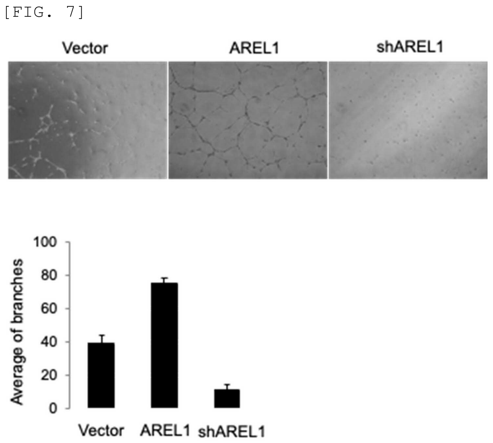

To investigate angiogenic activity, a HUVEC assay was performed. Compared to the control group, angiogenic activity of AREL1-expressing cells - capillary tube formation of HUVECs (vascular endothelial cells) increased. This indicates that AREL1 enhanced angiogenic activity, a key function of HIF-1α (see FIG. 7).

However, cells where AREL1 expression was inhibited by shAREL1 expression exhibited further reduced angiogenic activity compared to the control group. Therefore, this result shows that modulating AREL1 expression leads to either increased or decreased angiogenic activity. Therefore, these results demonstrate that AREL1 regulates HIF-1α *in vivo,* thereby modulating HIF-1α-dependent functions, including the transcriptional targets and angiogenesis.

The present invention described above is merely exemplary and those skilled in the art will easily appreciate that various modifications and equivalent other embodiments are possible. Therefore, it will be readily understood that the present invention is not limited to the embodiments disclosed in the detailed description above. Accordingly, the true technical protection scope of the present invention should be defined by the technical spirit of the appended claims. Furthermore, the present invention should be understood to include all modifications, equivalents, and alternatives within the spirit and scope of the present invention as defined by the appended claims.

## Claims

1. A HIF-1α activity modulator comprising, as an active ingredient, at least one of a compound, hexane, an antibody, and a peptide that regulates expression and activity of an AREL1 (apoptosis-resistant E3 ubiquitin protein ligase 1) gene or an AREL1 protein encoded by the AREL1 gene, or that interferes with interaction between the AREL1 gene and PHD2.

2. The HIF-1α activity modulator of claim 1, wherein the HIF-1α activity modulator is used for treatment, prevention, or diagnosis of at least one disease of autoimmune disease, ischemic/valvular heart disease, stroke, cancer, chronic lung disease, and congestive heart failure.

3. The HIF-1α activity modulator of claim 1, wherein the autoimmune disease comprises at least one of rheumatoid arthritis, alopecia areata, psoriasis, type 1 diabetes, hyperthyroidism, chronic thyroiditis, hypothyroidism, vitiligo, Crohn's disease, and autoimmune infection.
